# DEMANDE DE BREVET EUROPEEN

(11) **EP 4 806 447 A1**
(43) Date de publication de la demande: **16.09.2026**
(21) Numéro de dépôt: 26164641.8
(22) Date de dépôt: 13.03.2026
(51) Int. Cl.: A61K 35/745, A61K 35/747, A23L 33/135, A61K 31/592, A61K 31/593, A61K 31/7008, A61K 35/20, A61P 1/00, A61P 37/02, G01N 33/00

(54) **BACTÉRIES POUR LEUR UTILISATION CHEZ UN ENFANT ET/OU SA MÈRE, DANS LA PRÉVENTION OU LE TRAITEMENT DES EFFETS DÉLÉTÈRES APRÈS UNE DYSBIOSE DU MICROBIOTE EN ÂGE PRÉCOCE**

(30) Priorité: 14.03.2025 FR 2502594
(71) Demandeur: Larena, 49270 Oree D'Anjou (FR); Assistance Publique - Hôpitaux de Paris, 75012 Paris (FR); INSTITUT NATIONAL DE RECHERCHE POUR L'AGRICULTURE, L'ALIMENTATION ET L'ENVIRONNEMENT, 75007 Paris (FR); Institut National des Sciences et Industries du Vivant et de l'Environnement, 91120 Palaiseau (FR); Université Paris-Saclay, 91190 Gif-sur-Yvette (FR)
(72) Inventeur: DUBOURDEAUX, Michel, 03140 TAXAT (FR); HOLOWACZ, Sophie, 91160 SAULX LES CHARTREUX (FR); JACOUTON, Elsa, 92130 ISSY LES MOULINEAUX (FR); LENOIR, Loïc, 44400 REZÉ (FR); MARTIN ROSIQUE, Rebeca, 91430 IGNY (FR); LANGELLA, Philippe, 78140 VÉLIZY (FR); RIOS-COVIAN, David, 33011 OVIEDO (ES); MAITAN SANTOS, Bruna, 78350 JOUY-EN-JOSAS (FR); CHADI, Sead, 92160 ANTONY (FR); MOSCA, Alexis, 91400 ORSAY (FR)
(74) Mandataire: Jacobacci & Partners France

(57) **Abrégé**

La présente invention concerne des bactéries pour leur utilisation chez un enfant et/ou sa mère, au moins au cours de la période allant de la naissance dudit enfant jusqu'aux deux ans révolus dudit enfant, dans la prévention ou le traitement des effets délétères chez ledit enfant résultants de maladies non transmissibles qui sont observées, à moyen et/ou long termes, après une dysbiose du microbiote en âge précoce (correspondant à la primo-colonisation).

Ces bactéries sont choisies parmi les souches primo-colonisatrices d'un microbiote non dysbiotique, appartenant à un genre choisi parmi *Bifidobacterium* ou *Limosilactobacillus.*

## Description

### Domaine technique de l'invention

La présente invention concerne le domaine technique des bactéries pour leur utilisation chez un enfant et/ou sa mère.

### Etat de la technique

Certaines maladies non transmissibles (MNT), telles que les maladies inflammatoires chroniques de l'intestin (MICI), les maladies métaboliques et les allergies, peuvent être liées à des déséquilibres du microbiote intestinal survenant à un âge précoce chez l'enfant.

Ce déséquilibre, appelé « dysbiose », peut être causée par la naissance par césarienne, l'utilisation d'antibiotiques ou l'absence d'allaitement maternel.

Ces dysbioses perturbent la primo-colonisation du microbiote intestinal, impactant ainsi négativement la santé à long terme des enfants.

Les probiotiques sont couramment utilisés pour restaurer un microbiote équilibré chez les nourrissons. Cependant, les souches existantes ne sont pas toujours spécifiques aux besoins des enfants ayant subi une dysbiose précoce et montrent une efficacité variable.

La spécificité des souches, leur capacité à résister aux conditions gastriques et biliaires, ainsi que leur pouvoir immunomodulateur, sont des aspects critiques nécessitant une amélioration continue.

Il existe ainsi un besoin de développer des solutions probiotiques plus efficaces et spécifiques qui ciblent les dysbioses précoces et préviennent ou traitent les maladies non transmissibles associées.

De telles souches primo-colonisatrices spécifiques doivent être capables de restaurer la barrière intestinale, de réduire l'inflammation intestinale et de résister aux conditions physiologiques difficiles.

### Présentation de l'invention

Afin de remédier à l'inconvénient précité de l'état de la technique, la présente invention propose des bactéries spécifiques pour leur utilisation chez les enfants et/ou leurs mères, au moins durant la période allant de la naissance jusqu'à l'âge de deux ans, pour prévenir ou traiter les effets délétères des dysbioses du microbiote en âge précoce. Les souches proposées appartiennent à des genres tels que *Bifidobacterium* ou *Limosilactobacillus,* sélectionnées pour leurs propriétés uniques comme la réduction de l'inflammation intestinale, et la production d'acides gras à courte chaine (AGCC).

Plus particulièrement, on propose selon l'invention des bactéries pour leur utilisation chez un enfant et/ou sa mère, au moins au cours de la période allant de la naissance dudit enfant jusqu'aux deux ans révolus dudit enfant, dans la prévention ou le traitement des effets délétères chez cet enfant résultants de maladies non transmissibles qui sont observées, à moyen et/ou long termes, après une dysbiose du microbiote en âge précoce (correspondant à la primo-colonisation).

Les bactéries sont choisies parmi les souches primo-colonisatrices d'un microbiote non dysbiotique, appartenant à un genre choisi parmi :
- *Bifidobacterium,* de préférence parmi *Bifidobacterium longum,*
- *Limosilactobacillus,* par exemple parmi *Limosilactobacillus reuteri,*

Les bactéries correspondantes présentent les propriétés suivantes :
- une réduction de l'inflammation intestinale (avantageusement de 10 à 30%), mesurée in vitro par dosage de la cytokine inflammatoire IL-8 sur lignée épithéliale intestinale humaine,
- une production d'acétate, de préférence de 14 à 48 µmol/g,
et, de préférence encore :
- une résistance au pH acide, de préférence une perte inférieure à 0,3 log,
- une résistance aux sels biliaires, par exemple une perte de 0,07 à 3 log,
- une absence d'activité DNASE,
- une absence d'antibiorésistance.

L'invention propose ainsi une approche innovante pour la prévention ou le traitement des effets délétères observés à moyen et/ou long termes suite à une dysbiose du microbiote en âge précoce (correspondant à la primo-colonisation).

De manière générale, les bactéries spécifiques sélectionnées sont capables de renforcer et de restaurer la barrière intestinale, réduisant ainsi la perméabilité intestinale souvent compromise par une dysbiose. Cela aide à prévenir le passage des toxines et des pathogènes dans la circulation sanguine, réduisant les inflammations et autres complications associées.

Les souches probiotiques proposées possèdent des propriétés anti-inflammatoires qui peuvent réduire l'inflammation intestinale. En stabilisant l'environnement intestinal, elles aident à prévenir le développement de maladies inflammatoires chroniques comme les maladies inflammatoires de l'intestin (MICI).

Ces bactéries produisent encore des acides gras à chaîne courte (AGCC), tels que l'acide acétique (ou acétate), qui sont essentiels pour la santé colique. Les AGCC servent de source d'énergie pour les cellules épithéliales intestinales et ont des propriétés anti-inflammatoires, améliorant ainsi la santé globale du microbiote.

En outre, les souches sélectionnées sont spécifiquement adaptées pour les nourrissons et jeunes enfants. Elles sont choisies pour leur capacité à coloniser efficacement le microbiote intestinal des enfants, particulièrement après des perturbations comme la césarienne ou l'usage d'antibiotiques.

En particulier, les bactéries spécifiques selon l'invention permettent d'obtenir des effets bénéfiques significatifs (observés ici dans un modèle murin reproduisant une césarienne conventionnelle et reproduisant l'utilisation indiscriminée d'antibiotiques pendant la petite enfance et durant la grossesse, reflétant les conditions de dysbiose en âge précoce), à savoir : une réduction de la perméabilité intestinale, une réduction de la perte de poids, une réduction de l'inflammation et une protection contre le DNBS (modèle de colite).

Ces résultats mettent en évidence l'efficacité des bactéries pour rétablir un équilibre microbiotique sain et prévenir les conséquences à long terme des dysbioses en âge précoce.

Les bactéries sélectionnées comportent encore avantageusement des caractéristiques techniques complémentaires.

De préférence, les souches probiotiques sont sélectionnées pour leur capacité à résister aux conditions hostiles du tractus gastro-intestinal, incluant l'acidité gastrique et les sels biliaires. Cela garantit leur survie jusqu'à l'intestin où elles exercent leurs effets bénéfiques.

Les souches sont de préférence dépourvues d'activités DNASE, considérées comme un trait pathogène, et elles ne possèdent pas de gènes d'antibiorésistance, minimisant les risques de transfert de résistance aux antibiotiques, une préoccupation majeure en santé publique.

En stabilisant le microbiote dès les premiers jours de vie, l'invention contribue à un développement immunitaire optimal, réduisant le risque de maladies métaboliques, allergiques et inflammatoires à long terme. Cette intervention précoce favorise une meilleure santé générale tout au long de la vie.

D'autres caractéristiques non limitatives et avantageuses du produit/procédé conforme à l'invention, prises individuellement ou selon toutes les combinaisons techniquement possibles, sont les suivantes :
- lesdites bactéries sont isolées parmi les souches présentes dans les selles de nourrissons, lesdits nourrissons étant nés par voie basse et allaités, non médicamentés (en particulier en absence d'antibiotiques) et ayant moins de 3 jours, et dont leurs mères ne présentaient pas de maladie chronique, un IMC normal et n'ont pas été médicamentées, ni supplémentées en probiotiques le dernier trimestre de grossesse ;
- lesdites bactéries sont choisies parmi les bactéries disposant d'un statut Présomption d'innocuité reconnue (QPS) ;
- ladite période s'étend sur les 1000 premiers jours de l'enfant, depuis la grossesse jusqu'au deux ans révolus de l'enfant ;
- lesdites bactéries appartiennent aux souches choisies parmi la souche de l'espèce *L. reuteri* déposée auprès de la CNCM sous le numéro d'accession CNCM I-6110 le 11 septembre 2024, la souche de l'espèce *L. reuteri* déposée auprès de la CNCM sous le numéro d'accession CNCM I-6111 le 11 septembre 2024, la souche de l'espèce *L. reuteri* déposée auprès de la CNCM sous le numéro d'accession CNCM I-6112 le 11 septembre 2024, la souche de l'espèce *B. longum sbsp. longum* déposée auprès de la CNCM sous le numéro d'accession CNCM I-6114 le 11 septembre 2024 ;
- lesdites maladies non transmissibles sont choisies parmi les maladies chroniques, de préférence encore parmi les maladies inflammatoires chroniques de type MICI (maladie inflammatoire chronique de l'intestin), les maladies métaboliques, ou les allergies ;
- ladite dysbiose est survenue en âge précoce (de préférence dans une fenêtre des 1000er jours), éventuellement suite à au moins un facteur choisi parmi une naissance par voie césarienne, la prise d'antibiotiques, ou un allaitement artificiel.

La présente invention concerne encore une composition, par exemple sous la forme d'un complément alimentaire, d'une Denrée Alimentaire Destinée à des Fins Médicales Spéciales ou d'un produit infantile, comprenant au moins une bactérie selon l'invention, seule ou en combinaison, de préférence vivante.

La composition comprend de préférence au moins un ingrédient additionnel choisi parmi :
- une vitamine D,
- un prébiotique,
- les oligosaccharides du lait maternel (HMOs).

De préférence encore :
- ladite composition se présente sous une forme adaptée à l'administration par voie orale chez l'enfant, au cours de la période périnatale, par exemple sous forme de gouttes ou de sachets à diluer ;
- la composition, sous forme d'un complément alimentaire, adaptée à une utilisation non-thérapeutique au cours de la période périnatale chez l'enfant et/ou la mère, dans la prévention ou le traitement des effets délétères observés à moyen et/ou long termes suite à une dysbiose du microbiote en âge précoce (correspondant à la primo-colonisation).

La présente invention concerne encore un procédé pour la sélection de bactéries selon l'invention.

Ce procédé de sélection comprend les étapes successives suivantes :
- une étape de collecte, au cours de laquelle des selles de nourrissons sont collectés, lesdits nourrissons étant nés par voie basse et allaités, non médicamentés (en particulier en absence d'antibiotiques) et ayant moins de 3 jours, et dont leurs mères ne présentaient pas de maladie chronique, un IMC normal et n'ont pas été médicamentées, ni supplémentées en probiotiques le dernier trimestre de grossesse,
- une étape de sélection, au cours de laquelle ladite au moins une souche bactérienne est isolée et sélectionnée dans lesdits selles de nourrissons.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée en référence aux dessins qui illustrent des formes, non limitatives, de réalisation de l'invention et où :
[Fig. 1] est une vue de la mesure de la résistance au pH acide (pH 3), exprimée en Δlog CFU/mL ;
[Fig. 2] est une vue de la mesure de la résistance aux sels biliaires (0,3%), exprimée en Δlog CFU/mL ;
[Fig. 3] est une vue de la capacité anti-inflammatoire des souches, exprimée en pourcentage d'inhibition TNFα+ ;
   Légende : TNFα facteur de nécrose tumorale alpha. * p<0,05 ; ** p<0,01 ; **** p<0,0001 ;
[Fig. 4] est une vue de l'évolution du poids chez les souriceaux nés par césarienne ou voie vaginale ;
   Légende : VD : naissance par voie basse, CSD : naissance par césarienne, PBS: solution saline tamponnée au phosphate, DNBS : acide dinitrobenzène sulfonique. Test d'ANOVA à un facteur a été appliqué, suivi d'un test post hoc de Fisher sans correction pour comparaisons multiples. Les valeurs sont exprimées en moyenne ± ESM. ** p<0,01 ;
   a) 3 semaines - poids corporel en g, b) 6 semaines - poids corporel en g, c) 6 semaines (après modèle de colite - DNBS) - AUC (attendu vs. observé ;

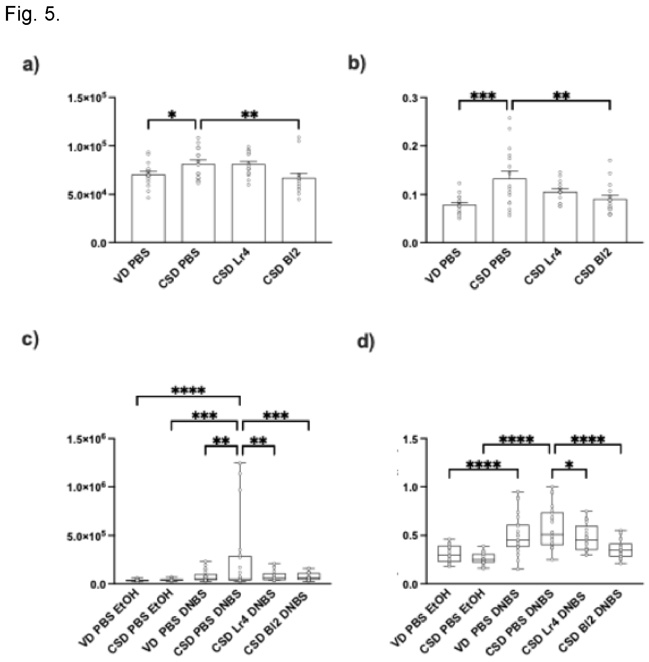

[Fig. 5] est une vue du suivi de l'inflammation systémique, locale et de la perméabilité systémique des souriceaux nés par voies vaginales ou césariennes ;
   Légende : VD : naissance par voie basse, CSD : naissance par césarienne, DNBS : acide dinitrobenzène sulfonique, PBS: solution saline tamponnée au phosphate, EtOH: PBS éthanol 30%, LPN2: lipocaline 2. Un test d'ANOVA à un facteur a été appliqué, suivi d'un test post hoc de Fisher sans correction pour comparaisons multiples, afin d'évaluer les différences des niveaux plasmatiques de sCD14 (Fig. 5a) et de lipocaline 2 (LPN2; Fig. 5b) entre les groupes chez les animaux âgés de 3 semaines. Une analyse à effets mixtes, suivie d'un test post hoc de Fisher sans correction pour comparaisons multiples, a été réalisée pour évaluer les différences des niveaux plasmatiques de sCD14 (Fig. 5c) et de l'épaisseur de la paroi colique (Fig. 5d) entre les groupes âgés de 6 semaines, avec ou sans colite DNBS). Les valeurs sont exprimées en moyenne ± ESM. * p < 0,05; ** p < 0,01;*** p < 0,001; **** p < 0,0001 ;
   a) 3 semaines - niveaux plasmatiques de sCD14 (pg/ml), b) 3 semaines - niveaux plasmatiques de LPN 2 (mg/ml), c) 6 semaines (après modèle de colite - DNBS) - niveaux plasmatiques de sCD14 (pg/ml), d) 6 semaines (après modèle de colite - DNBS) - l'épaisseur de la paroi du côlon (µm) ;

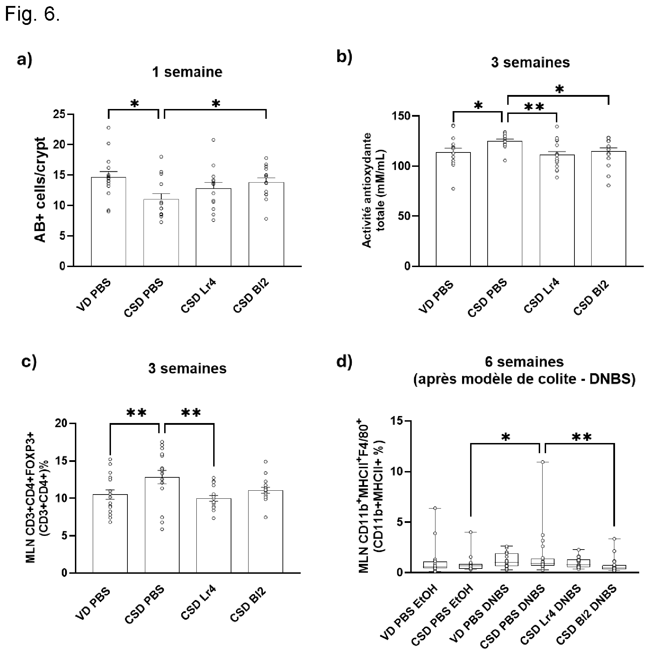

[Fig. 6] est une vue du suivi de la supplémentation en probiotique a) sur les cellules caliciformes positives à la mucine acide par crypte colique en semaine 1, b) sur l'activité totale antioxydante, c) sur le niveau de cellules T régulatrices dans les ganglions mésentériques en semaine 3, d) et sur le niveau de cellules macrophagiques dans les ganglions mésentériques en semaine 6.
   Légende : VD : naissance par voie basse, CSD : naissance par césarienne, PBS: solution saline tamponnée au phosphate, DNBS : acide dinitrobenzène sulfonique. MLN : mesenteric lymphe node/ ganglions mésentériques. Un test ANOVA à un facteur a été appliqué suivi d'un test post hoc de Fisher sans correction pour comparaisons multiples si les données suivent une distribution normale. Le cas échéant un test de Kruskal Wallis suivi d'un test post hoc de Dunn, * p<0,05 ; ** p<0,01;

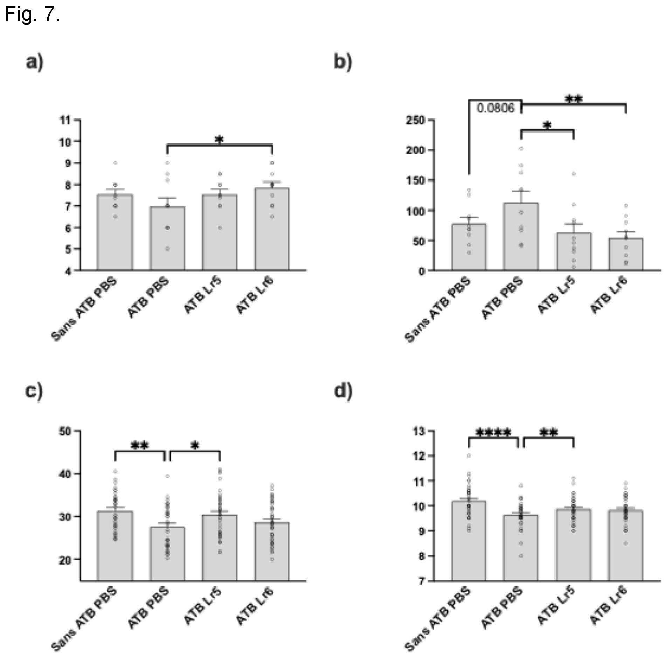

[Fig. 7] est une vue du suivi de l'inflammation locale et systémique, ainsi que du poids et de la longueur corporelle des souriceaux soumis à un traitement antibiotique précoce ;
   Légende : ATB : antibiotiques, LPN2 : lipocaline 2. Un test ANOVA à un facteur a été appliqué pour les animaux âgés de 4 semaines (Fig. 7a-b), et une analyse à effets mixtes a été réalisée pour les animaux âgés de 6 semaines (Fig. 7c-d), suivies d'un test post hoc de Fisher sans correction pour comparaisons multiples. Les valeurs sont exprimées en moyenne ± ESM. * p<0,05; ** p<0,01; **** p<0,0001 ;
   a) 4 semaines (femelles) - longueur du côlon en cm ; b) 4 semaines - niveaux plasmatiques de LPN 2 exprimés en µg/ml ; c) 6 semaines - poids corporel exprimé en g ; d) 6 semaines - longueur du corps exprimée en cm ;

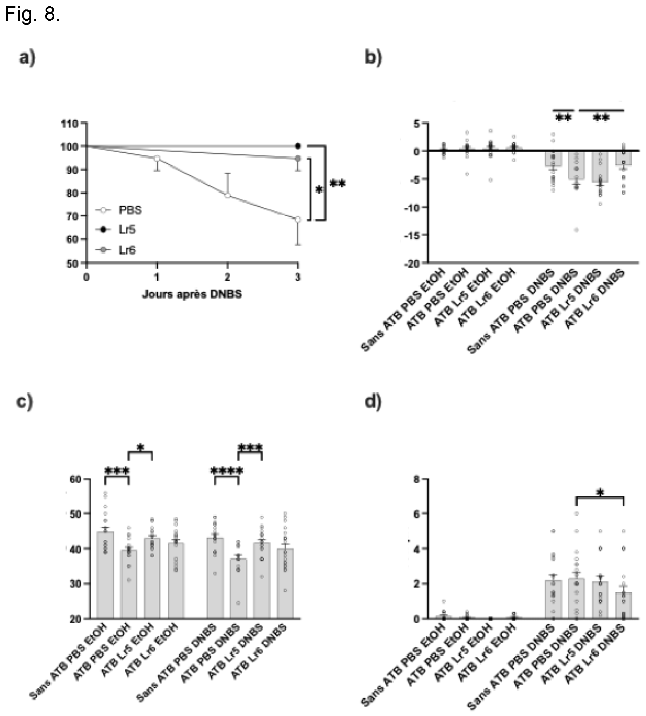

[Fig. 8] est une vue de la survie de souriceaux soumis à un traitement par antibiotiques précoce puis à une colite induite par DNBS ;
   Légende : ATB : antibiotiques, PBS: solution saline tamponnée au phosphate, EtOH: PBS éthanol 30%. Une analyse à effets mixtes a été réalisée, suivies d'un test post hoc de Fisher sans correction pour comparaisons multiples. Les valeurs sont exprimées en fraction de survie ± ES dans la Fig. 8a, et en moyenne ± ESM dans les Fig. 8b-d. * p<0,05; ** p<0,01; *** p<0,001; **** p<0,0001 ;
   a) 6 semaines (modèle de colite - DNBS) - pourcentage de survie des animaux ATB DNBS ; b) 6 semaines (modèle de colite - DNBS) - delta poids corporel en g ; c) 6 semaines (modèle de colite - DNBS) - longueur de l'intestin grêle en cm ; d) 6 semaines (modèle de colite - DNBS) - score macroscopique colique.

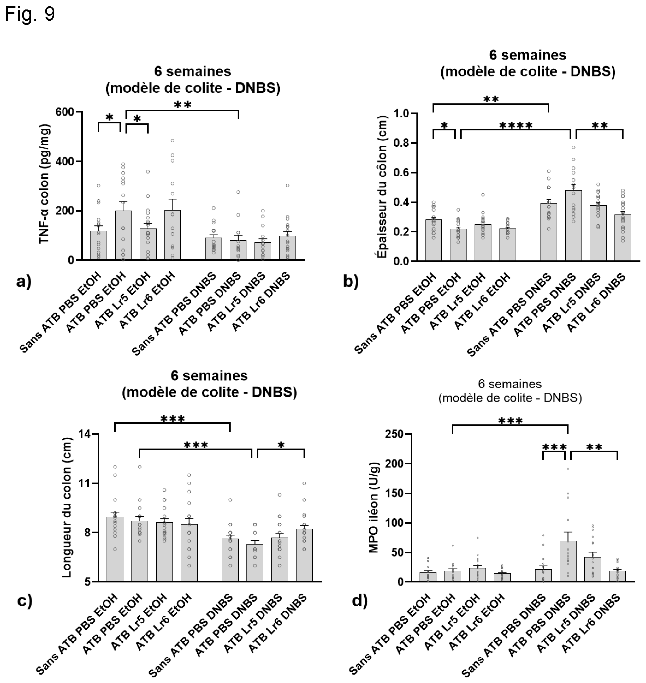

[Fig. 9] est une vue de l'effet de la supplémentation en probiotiques en semaine 6 avant et après challenge au DNBS sur les paramètres inflammatoires locaux, a) niveau de TNF-a dans le colon, b) épaisseur du colon, c) longueur du colon et du niveau de MPO dans l'iléon.

Légende : ATB : antibiotiques, EtOH: PBS éthanol 30%, VD : naissance par voie basse, CSD : naissance par césarienne, PBS: solution saline tamponnée au phosphate, DNBS : acide dinitrobenzène sulfonique. TNF-α : facteur de nécrose tumorale alpha, MPO : myélopéroxydase. Un test ANOVA à un facteur a été appliqué suivi d'un test post hoc de Fisher sans correction pour comparaisons multiples si les données suivent une distribution normale. Le cas échéant un test de Kruskal Wallis suivi d'un test post hoc de Dunn * p<0,05 ; ** p<0,01 ; *** p<0,001 ; **** p<0,0001.

### Bactéries

La présente invention concerne ainsi des bactéries spécifiques, pour leur utilisation chez un enfant et/ou sa mère, au moins au cours de la période allant de la naissance dudit enfant jusqu'aux deux ans révolus dudit enfant.

Selon un mode de réalisation préféré, la période s'étend sur les 1000 premiers jours de l'enfant, depuis la grossesse jusqu'au deux ans révolus de l'enfant.

Par « bactéries », on entend avantageusement des micro-organismes vivants, appelés également « probiotiques » ou « souches probiotiques », qui confèrent un bénéfice pour la santé de l'hôte lorsqu'ils sont administrés en quantités adéquates.

Ces bactéries bénéfiques, appartenant aux genres *Bifidobacterium* ou *Limosilactobacillus,* sont spécifiquement sélectionnées pour leurs propriétés de colonisation du microbiote intestinal, leur capacité à améliorer la santé intestinale, réduire les inflammations, et favoriser un équilibre microbien sain.

Selon l'invention, ces souches sont choisies pour leur aptitude à coloniser le microbiote intestinal, assurant ainsi une primo-colonisation optimale et contribuant à la prévention ou au traitement des effets délétères chez l'enfant, résultants de maladies non transmissibles qui sont observées après une dysbiose du microbiote en âge précoce.

Par « dysbiose du microbiote en âge précoce », on entend avantageusement un déséquilibre dans la composition du microbiote intestinal qui se produit au cours des premiers jours ou mois de vie.

De préférence, une telle dysbiose est survenue en âge précoce (de préférence dans une fenêtre des 1000er jours), éventuellement suite à au moins un facteur choisi parmi :
- une naissance par voie césarienne, ou
- la prise d'antibiotiques, ou
- un allaitement artificiel.

De manière générale, la dysbiose en âge précoce n'est pas une condition nécessitant un diagnostic par des moyens complexes, mais est identifiée par la présence de facteurs de risque bien connus survenant durant la période périnatale.

Plus généralement, le diagnostic d'une dysbiose, telle que définie dans la présente invention, peut reposer sur l'analyse approfondie de la composition, de la diversité et de la fonctionnalité des micro-organismes présents dans l'intestin.

Cette identification permet de cibler précisément les populations nécessitant l'administration des bactéries de l'invention.

Plusieurs méthodes et indicateurs scientifiques et cliniques peuvent être mis en œuvre.
1. Technologies d'analyse génétique et moléculaire. Ces technologies permettent de cartographier précisément le microbiote intestinal pour identifier les écarts par rapport à un microbiote non dysbiotique.

Le séquençage de nouvelle génération (NGS) : Cette méthode de référence permet de s'affranchir des limites de la culture traditionnelle.

Elle comprend notamment :
- Le séquençage du gène de l'ARNr 16S : Utilisé pour identifier les espèces présentes et mesurer leur abondance relative. Il cible des régions hypervariables spécifiques aux bactéries.
- Le séquençage métagénomique (Shotgun) : Permet de séquencer l'intégralité des génomes microbiens d'un échantillon, incluant les virus et les champignons, pour comprendre les capacités fonctionnelles et métaboliques globales du microbiote.

Les techniques « omiques » fonctionnelles : La métabolomique analyse les métabolites produits par le microbiote, tels que les acides gras à chaîne courte (AGCC).

2. Indicateurs de déséquilibre (Marqueurs de Dysbiose). L'analyse des données moléculaires permet d'établir des signatures typiques de la dysbiose précoce:
- Le Microbiome Health Index (MHI) : Indice évaluant l'état de santé du microbiote par l'équilibre entre les groupes *Firmicutes, Bacteroidetes* et *Proteobacteria.*
- La perte de diversité : Quantifiée par des mesures mathématiques telles que l'indice de Shannon, une diminution de la diversité globale est un indicateur fort de dysbiose.
- Le ratio des espèces : Une réduction de 10% à 40% des bactéries bénéfiques (telles que *Bifidobacterium* et *Limosilactobacillus*) au profit de pathogènes opportunistes (comme les *Proteobacteria,* dont le taux peut passer de moins de 10% à plus de 20-30%) signe un état pathologique. Le ratio *Bacteroidetes*/*Firmicutes* est également une caractéristique technique surveillée.

3. Évaluation clinique et marqueurs indirects. Conformément aux maladies non transmissibles visées par l'invention, le diagnostic peut être complété par :
- L'analyse fécale de la calprotectine : Protéine dont le taux augmente lors d'une inflammation du tube digestif, comme dans le cas des MICI.
- Les bilans sanguins : Dosage de la protéine CRP pour repérer une inflammation systémique.
- L'historique périnatal : L'exposition à des facteurs de risque tels que la naissance par césarienne ou la prise d'antibiotiques constitue un critère de diagnostic présumé de dysbiose en âge précoce.

La présente invention concerne ainsi avantageusement ces bactéries pour leur utilisation chez un enfant :
- ayant été diagnostiqué comme souffrant, ou ayant souffert, d'une dysbiose, ou
- identifié comme étant à risque de dysbiose.

Par « enfant diagnostiqué comme souffrant d'une dysbiose », on entend avantageusement un enfant chez qui une analyse moléculaire (par exemple NGS), fonctionnelle (par exemple faible production d'acétate) ou clinique (par exemple calprotectine fécale élevée) a confirmé un déséquilibre de la composition ou de la diversité du microbiote intestinal par rapport à un microbiote non dysbiotique.

Par « ayant souffert d'une dysbiose », on entend avantageusement un état de déséquilibre du microbiote intestinal survenu durant la période de primo-colonisation, dont la phase aiguë est potentiellement résolue.

Cette phase peut avoir laissé des altérations fonctionnelles ou structurelles (telles qu'une barrière intestinale affaiblie ou une inflammation persistante) susceptibles de générer des maladies non transmissibles à moyen ou long terme.

Cette dysbiose perturbe la primo-colonisation du microbiote, susceptible d'entraîner des effets délétères à moyen et long terme sur la santé.

Par « maladies non transmissibles qui sont observées, à moyen et/ou long termes, après une dysbiose du microbiote en âge précoce », on entend en particulier des maladies ou affections chroniques.

Parmi ces maladies chroniques, on englobe en particulier :
- les maladies inflammatoires chroniques de type MICI (maladie inflammatoire chronique de l'intestin),
- les maladies métaboliques, tel que le diabète, ou
- les allergies, ou
- toutes autres maladies liées à une perturbation précoce du microbiote intestinal.

Les maladies inflammatoires chroniques de l'intestin (ou MICI) regroupent la maladie de Crohn (MC) et la rectocolite hémorragique (RCH). Toutes deux se caractérisent par une inflammation de la paroi d'une partie du tube digestif, due à une dérégulation du système immunitaire intestinal.

Par « maladies métaboliques », on entend avantageusement des affections chroniques qui perturbent les processus métaboliques de l'organisme. Ces maladies incluent des conditions comme le diabète, l'obésité, et les dyslipidémies, caractérisées par des déséquilibres dans la régulation de la glycémie, des lipides, et d'autres aspects du métabolisme. Ces maladies peuvent entraîner diverses complications graves, telles que des maladies cardiovasculaires, des lésions nerveuses, et des dysfonctionnements rénaux, nécessitant une gestion continue et une intervention médicale pour prévenir ces effets délétères à long terme. Ces maladies peuvent apparaitre suite à une dysbiose en âge précoce, affectant durablement la santé en raison de la primo-colonisation inadéquate du microbiote intestinal.

Par « allergies », on entend avantageusement des réactions immunitaires anormales et excessives à des substances généralement inoffensives, appelées allergènes. Ces réactions peuvent inclure des symptômes tels que des éruptions cutanées, des démangeaisons, des gonflements, des difficultés respiratoires, et des réactions anaphylactiques graves. Les allergènes courants incluent les pollens, les acariens, certains aliments, le venin d'insectes, et des médicaments. Les allergies peuvent avoir un impact significatif sur la qualité de vie et nécessitent souvent une gestion continue pour prévenir et traiter les symptômes.

Par « moyen et/ou long termes », on entend avantageusement les périodes de temps qui s'étendent respectivement sur plusieurs mois à quelques années (moyen terme) et sur plusieurs années à des décennies (long terme). Dans le contexte des effets de la dysbiose du microbiote en âge précoce, cela fait référence aux impacts sur la santé de l'enfant qui peuvent apparaître et persister à partir de quelques mois après la naissance jusqu'à l'âge adulte.

Le moyen terme, englobant l'enfance et l'adolescence, s'étend avantageusement de la fin de la primo-colonisation jusqu'à la fin de la puberté, correspond à la phase de croissance et de maturation fonctionnelle du microbiote.

Le long terme, de préférence âge adulte et vie entière, désigne avantageusement l'impact persistant sur plusieurs années ou décennies, affectant la santé globale de l'individu à l'âge adulte.

En conclusion, l'expression « observées à moyen et/ou long termes » désigne avantageusement l'apparition de maladies non transmissibles pendant l'enfance, l'adolescence (moyen terme) ou à l'âge adulte (long terme), soit la période de la vie succédant à l'âge de 3 ans, âge auquel le microbiote a achevé sa phase critique de primo-colonisation et de stabilisation.

Par « effets délétères chez l'enfant », on entend en particulier les conséquences négatives sur la santé de l'enfant qui découlent des maladies précitées.

Ces effets incluent des symptômes tels que des inflammations persistantes, des déséquilibres métaboliques, des réactions allergiques sévères, des troubles digestifs, et des altérations du développement immunitaire, survenant après une dysbiose du microbiote en âge précoce.

Par « prévention », on entend avantageusement l'ensemble des mesures prises pour empêcher l'apparition des effets délétères chez l'enfant résultant de ces maladies non transmissibles. Cela inclut l'utilisation des souches probiotiques spécifiques dès les premiers jours de vie pour favoriser un microbiote sain, réduisant ainsi le risque de dysbiose et ses conséquences à long terme sur la santé.

Par « traitement », on entend avantageusement les interventions visant à atténuer ou éliminer les effets délétères chez l'enfant résultant de ces maladies non transmissibles après l'apparition de la dysbiose. Cela inclut l'administration de probiotiques pour rééquilibrer le microbiote et réduire les symptômes ou complications associées aux dysbioses précoces.

Selon l'invention, les bactéries sont avantageusement choisies parmi les souches primo-colonisatrices d'un microbiote non dysbiotique, appartenant à un genre choisi parmi :
- *Bifidobacterium,* de préférence parmi *Bifidobacterium longum,*
- *Limosilactobacillus,* par exemple parmi *Limosilactobacillus reuteri,*

Par « souches primo-colonisatrices d'un microbiote non dysbiotique », on entend avantageusement des bactéries spécifiques sélectionnées pour leur capacité à établir un microbiote équilibré et sain dès les premiers jours de vie. Ces souches favorisent une colonisation initiale optimale du microbiote intestinal, empêchant le développement d'une dysbiose. Elles jouent un rôle crucial dans la maturation du système immunitaire et le maintien de la santé intestinale à long terme.

Ces bactéries spécifiques présentent les propriétés suivantes :
- une réduction (de préférence de 10 à 30 %) de l'inflammation intestinale, mesurée in vitro par dosage de la cytokine inflammatoire IL-8 sur lignée épithéliale intestinale humaine,
- une production d'acétate, de préférence de 14 à 48 µmol/g.

Par « une réduction de l'inflammation intestinale, mesurée in vitro par dosage de la cytokine inflammatoire IL-8 sur lignée épithéliale intestinale humaine », on entend avantageusement la capacité des souches probiotiques à diminuer la production de la cytokine IL-8, un marqueur clé de l'inflammation.

Cette réduction est évaluée en utilisant des cellules épithéliales humaines (par exemple des cellules HT-29), où une diminution des niveaux d'IL-8 indique une réduction de la réponse inflammatoire, contribuant ainsi à un environnement intestinal plus sain.

En l'espèce, la diminution de la production de la cytokine IL-8 est avantageusement d'au moins 10%.

La mesure de production de cytokine IL-8 est par exemple décrite dans les documents suivantes :
- Microorganisms. 2023 Mar 30;11(4):906. doi: 10.3390/microorganisms11040906,
- Front Microbiol. 2023 Nov 29;14:1270974. doi: 10.3389/fmicb.2023.1270974.

Par « une production d'acétate », on entend avantageusement la capacité des souches probiotiques à fermenter les fibres alimentaires non digestibles dans l'intestin, produisant ainsi des acides gras bénéfiques.

Les AGCCs sont avantageusement choisis parmi l'acétate.

Ces AGCC jouent un rôle crucial dans la santé intestinale en fournissant de l'énergie aux cellules coliques, en renforçant la barrière intestinale, et en possédant des propriétés anti-inflammatoires, contribuant ainsi à un environnement intestinal équilibré et sain.

De préférence, la production d'acétate présente une valeur allant de 10 à 80 µmol/g.

La production d'acétate est par exemple déterminée par le procédé décrit dans Microorganisms. 2023 Mar 30;11(4):906. doi: 10.3390/microorganisms11040906.

De préférence, ces bactéries présentent encore les propriétés suivantes :
- une résistance au pH acide (avantageusement une perte < 0,3 log),
- une résistance aux sels biliaires (avantageusement une perte de 0,07 log à 3 log),
- une absence d'activité DNASE,
- une absence d'antibiorésistance.

Par « résistance au pH acide », on entend avantageusement la capacité des souches probiotiques à survivre et maintenir leur viabilité dans les conditions acides de l'estomac, garantissant ainsi leur passage vers l'intestin où elles exercent leurs effets bénéfiques.

En pratique, la valeur recherchée est un ratio de 40% de viabilité pour les unités formatrices de colonies (UFC) en condition stressée (pH 3 pendant 1h à 37°C) *versus* condition contrôle (sans stress).

Par « absence d'activité DNASE », on entend avantageusement que les souches probiotiques ne possèdent pas l'enzyme DNASE.

Par « absence d'antibiorésistance », on entend avantageusement que les souches probiotiques ne possèdent pas de gènes de résistance aux antibiotiques, minimisant ainsi le risque de transfert de ces résistances à d'autres bactéries pathogènes, contribuant à la lutte contre l'antibiorésistance.

En pratique, les bactéries d'intérêt sont en particulier sensibles à la gentamycine, streptomycine, clindamycine, chloramphénicol, érythromycine et la tétracycline (sauf la souche Lr4). Seule la souche BI2 est sensible à l'ampicilline et à la vancomycine. Les souches de *Limosilactobacillus reuteri* sont sensibles à la kanamycine.

Selon encore un mode de réalisation préféré, les bactéries sont choisies parmi les bactéries disposant d'un statut Présomption d'innocuité reconnue (QPS).

Les bactéries disposant d'un statut « Présomption d'innocuité reconnue (QPS) » sont reconnues comme sûres par l'Autorité européenne de sécurité des aliments (EFSA).

Le statut QPS attribue une présomption d'innocuité basée sur l'identité taxonomique, le corpus de connaissances, et l'absence de problèmes de sécurité potentiels.

Ce processus facilite une évaluation accélérée des souches microbiennes pour leur mise sur le marché, garantissant leur sécurité pour l'homme, les animaux et l'environnement.

De manière générale, les bactéries appartiennent avantageusement à :
- la souche de l'espèce *L. reuteri* déposée auprès de la CNCM sous le numéro d'accession CNCM I-6110 le 11 septembre 2024,
- la souche de l'espèce *L*. *reuteri* déposée auprès de la CNCM sous le numéro d'accession CNCM I-6111 le 11 septembre 2024,
- la souche de l'espèce *L*. *reuteri* déposée auprès de la CNCM sous le numéro d'accession CNCM I-6112 le 11 septembre 2024,
- la souche de l'espèce *B*. *longum sbsp. longum* déposée auprès de la CNCM sous le numéro d'accession CNCM I-6114 le 11 septembre 2024.

En pratique et selon un mode de réalisation préféré, les bactéries sont isolées parmi les souches présentes dans les selles de nourrissons

Par « isolées parmi les souches présentes dans les selles de nourrissons », on entend avantageusement des bactéries sélectionnées spécifiquement à partir des micro-organismes trouvés dans les échantillons de selles de bébés en bonne santé.

Par « bébés en bonne santé », on entend avantageusement des nourrissons qui présentent un développement physique, mental et social optimal. Cela inclut une croissance appropriée aux courbes de développement, une absence de maladies ou d'infirmités et un allaitement maternel.

Les nourrissons sont nés par voie basse et allaités, non médicamentés (en particulier en absence d'antibiotiques) et ayant moins de 3 jours.

Leurs mères ne présentent pas de maladie chronique, un IMC normal et n'ont pas été médicamentées, ni supplémentées en probiotiques le dernier trimestre de grossesse.

Par « IMC normal », on entend avantageusement un indice de masse corporelle (IMC) se situant dans une plage considérée comme saine pour la taille et le poids d'un individu. L'IMC est calculé en divisant le poids en kilogrammes par la taille en mètres carrés (kg/m²). Pour les adultes, un IMC normal se situe généralement entre 18,5 et 24,9.

Par « pas été médicamentées », on entend avantageusement des femmes enceintes ou allaitantes qui n'ont pas reçu de traitements médicaux pharmacologiques, tels que des antibiotiques, des antiviraux, des antifongiques, ou autres médicaments pendant la période de grossesse ou d'allaitement.

En d'autres termes, les critères d'inclusion des nourrissons sont :
- nourrissons nés à terme, soit au moins à 37 semaines de grossesse,
- un allaitement maternel exclusif,
- une absence de traitement antibiotique intrapartum,
- une absence de prise d'antibiotiques par la mère au troisième trimestre de grossesse,
- une absence de prise de probiotiques par la mère au troisième trimestre de grossesse,
- une absence de pathologie chronique chez la mère,
- une absence de traitement chronique chez la mère,
- un indice de masse corporelle (IMC) maternel inférieur à 25 en début de grossesse,
- une absence de diabète gestationnel,
- une absence de pathologies chez le nourrisson, et
- une absence de supplémentation médicale (autre que les vitamines conventionnelles).

### Composition

La présente invention concerne encore la composition comprenant les bactéries selon l'invention, de préférence vivante.

De manière générale, les bactéries vivantes, lorsqu'elles sont ingérées en quantité suffisante, exercent des effets positifs sur la santé, au-delà des effets nutritionnels traditionnels.

Par forme « vivante », on entend une forme dotée de la capacité à se multiplier sous réserve de la placer dans un environnement propice à la récupération de cette capacité.

Ainsi, au sens de la présente invention, le terme vivant couvre l'état dit dormance dans lequel les microorganismes peuvent être placés suite à un traitement physicochimique tel que par exemple la lyophilisation.

De manière générale, la souche probiotique est avantageusement mise en œuvre sous une forme lyophilisée.

Ce type de formulation possède les avantages d'être facilement accessible, de mise en œuvre aisée et de ne soulever aucune difficulté et/ou contrainte en termes de stockage. Par ailleurs, il est compatible avec le conditionnement de microorganismes à l'état de dormance.

Cette lyophilisation peut être réalisée selon des méthodes conventionnelles ou selon une méthode décrite par exemple dans le document FR-3 103 827.

La concentration en souche probiotique, dans la composition, est par exemple de 1x10⁹ CFU par dose quotidienne.

La concentration en souche probiotique dans la composition peut être d'une manière générale une quantité efficace permettant d'obtenir l'effet recherché. Cette quantité peut être déterminée par la personne du métier au moyen de ses connaissances générales et par des tests usuels. Cette quantité peut par exemple être, en dose journalière exprimée en CFU, au minimum d'environ 1x10⁹.

Elle peut être alternativement d'environ 2,5x10⁹ CFU/g de produit.

La souche probiotique peut encore être incluse à hauteur de 15 à 20% dans la composition (poids/poids).

La souche probiotique convenant à l'invention est physiologiquement acceptable. En d'autres termes, cette souche probiotique peut être administrée sans risques.

Une telle composition comprend avantageusement un mélange spécifique de divers composants formant un produit fini.

Dans le contexte des probiotiques, la composition selon l'invention comprend avantageusement :
- les souches bactériennes selon l'invention,
- des excipients,
- éventuellement des agents stabilisants nécessaires pour assurer la viabilité des bactéries et l'efficacité du produit.

La composition selon l'invention comprend de préférence encore au moins un ingrédient additionnel choisi parmi :
- une vitamine D,
- un prébiotique,
- les oligosaccharides du lait maternel (HMOs).

Les prébiotiques incluent avantageusement des substances alimentaires non digestibles qui favorisent la croissance et l'activité des bactéries bénéfiques dans le microbiote intestinal. Les prébiotiques servent de nourriture pour les probiotiques, aidant ainsi à maintenir et à améliorer la santé intestinale. Ils incluent souvent des fibres alimentaires telles que les fructo-oligosaccharides (FOS), les galacto-oligosaccharides (GOS), l'inuline, et autres polysaccharides non digestibles. Les prébiotiques contribuent à l'équilibre du microbiote en stimulant sélectivement la croissance des bactéries bénéfiques.

Les oligosaccharides du lait maternel (HMOs) sont des composés glucidiques complexes présents dans le lait maternel humain. Ces oligosaccharides ne sont pas digestibles par l'intestin humain mais servent de substrat pour les bactéries bénéfiques dans le microbiote intestinal du nourrisson. Les HMOs jouent un rôle crucial dans le développement du système immunitaire, la prévention des infections et l'établissement d'un microbiote intestinal sain en favorisant la croissance de souches probiotiques spécifiques.

De préférence, la composition se présente sous une forme choisie parmi :
- un complément alimentaire,
- une Denrée Alimentaire Destinée à des Fins Médicales Spéciales ou
- un produit infantile.

Un complément alimentaire forme avantageusement une préparation destinés à compléter l'alimentation normale et à fournir des nutriments supplémentaires qui peuvent être insuffisants ou absents dans l'alimentation d'une personne.

Dans ce cadre, l'invention concerne encore la composition selon l'invention, sous forme d'un complément alimentaire, adaptée à une utilisation non-thérapeutique au cours de la période périnatale chez l'enfant et/ou la mère, dans la prévention ou le traitement des effets délétères observés à moyen et/ou long termes suite à une dysbiose du microbiote en âge précoce (correspondant à la primo-colonisation).

Par « Denrée Alimentaire Destinée à des Fins Médicales Spéciales (DADFMS) », on entend avantageusement une composition alimentaire spécialement formulée pour la prise en charge diététique de patients, incluant des nourrissons et des jeunes enfants, atteints de troubles spécifiques, résultant notamment de dysbioses du microbiote en âge précoce. Cette composition est utilisée sous supervision médicale et est conçue pour répondre à des besoins nutritionnels particuliers que l'alimentation normale ne peut satisfaire.

Par « produit infantile », on entend avantageusement des aliments spécialement conçus pour répondre aux besoins nutritionnels des nourrissons et des jeunes enfants.

En pratique, la composition se présente avantageusement sous une forme adaptée à l'administration par voie orale chez l'enfant, au cours de la période périnatale, par exemple sous forme de gouttes ou de sachets à diluer.

### Procédé de sélection

La présente invention concerne encore le procédé pour la sélection de bactéries selon l'invention.

Ce procédé de sélection comprend une étape de collecte, au cours de laquelle des selles de nourrissons sont collectés (par exemple issues des couches de bébés).

Les nourrissons sont nés par voie basse et allaités, non médicamentés (en particulier en absence d'antibiotiques) et ayant moins de 3 jours, et dont leurs mères ne présentaient pas de maladie chronique, un IMC normal et n'ont pas été médicamentées, ni supplémentées en probiotiques le dernier trimestre de grossesse.

Ce procédé comprend ensuite une étape de sélection, au cours de laquelle ladite au moins une souche bactérienne est isolée et sélectionnée dans ces selles de nourrissons.

Par exemple, cette étape de sélection comprend les opérations suivantes de préparation des échantillons :
- les matières fécales de nourrissons, collectées dans un environnement hospitalier, sont conservées dans des conditions anaérobies pour maintenir la viabilité des bactéries jusqu'à leur traitement,
- les échantillons fécaux sont dilués dans une solution tamponnée (PBS) et des dilutions en série sont réalisées,
- les dilutions sont ensuite étalées sur des boîtes de Pétri contenant divers milieux de culture pour maximiser la diversité des souches isolées (par exemple, MRS, GAMc, RCM, BEAA),
- les colonies bactériennes isolées sont transférées dans des milieux liquides appropriés (MRSc ou GAMc) et après plusieurs passages, elles sont identifiées par séquençage du gène de l'ARNr 16s pour déterminer leurs caractéristiques spécifiques et probiotiques.

Le procédé de sélection comprend encore des opérations suivantes de détermination qualitative :
- mesure in vitro par dosage de la cytokine inflammatoire IL-8 sur lignée épithéliale intestinale humaine,
- mesure de la production d'acétate,
et, de préférence encore :
- mesure de la résistance au pH acide,
- mesure de la résistance aux sels biliaires,
- mesure de l'absence d'activité DNASE,
- mesure de l'absence d'antibiorésistance.

Bien entendu, diverses autres modifications peuvent être apportées à l'invention dans le cadre des revendications annexées.

### Exemples

### Exemple 1

### 1. Matériel et Méthode

### Isolement et identification bactérienne :

Les matières fécales (issues des couches de bébés nés par voie basse à l'âge de 1 ou 2 jours et collectées à l'hôpital Robert Debré) ont été déposées dans un dispositive de prélèvements « GutAlive » (Microviable Therapeutics, Espagne), afin de maintenir des conditions anaérobies de la collecte au traitement des échantillons. Les échantillons ont ensuite été manipulés en chambre anaérobie.

Les matières fécales ont été diluées à 1 : 9 dans du PBS et des dilutions en série ont été effectuées. Des dilutions ont ensuite été étalées sur des boites de pétri contenant plusieurs milieux afin d'augmenter la diversité des souches isolées.

Les milieux utilisés étaient : MRS (BD), GAMc (Yaku, Japon), RCM (MERK) et BEAA (Biokar).

Les colonies ont ensuite été transférées dans des MRSc ou des GAMc liquides et, après 2 passages, identifiées par séquençage du gène de l'ARNr 16s.

**[Tableau 1]**

| **Espèces** | **Code CNCM** | **Code LA** | **Code** | **Milieux** | **Condition O₂** | **Température** |
|---|---|---|---|---|---|---|
| *Limosilactobacillus reuteri* | ***CNCM I-6110*** | LA 710 | Lr4 | MRSc | Aérobie | 37°C |
| *Limosilactobacillus reuteri* | ***CNCM I-6111*** | LA 711 | Lr5 | MRSc | Anaérobie | 37°C |
| *Limosilactobacillus reuteri* | ***CNCM I-6112*** | LA 712 | Lr6 | MRSc | Aérobie | 37°C |
| *Bifidobacterium longum sbsp. longum* | ***CNCM I-6114*** | LA 714 | BI2 | MRSc | Anaérobie | 37°C |

### Liste des souches et conditions de cultures

### Resistance aux conditions gastro-intestinales :

Les souches ont été récoltées après une croissance d'une nuit dans leur milieux respectifs, centrifugées et resuspendues dans 1 ml du PBS (OD₆₀₀ nm = 0.1) supplémentés avec des sels biliaires à 0,3% (Bile bovine, Sigma B3883) ou avec un pH acide (pH=3) et mises en culture pendant 1 h à 37°C (conditions stressés).

Un contrôle est mené en parallèle avec seulement du PBS. Les solutions bactériennes ont ensuite été ensemencées sur géloses pour dénombrement. Les résultats sont exprimés selon le ratio des unités formant des colonies (UFC) en condition stressée *versus* condition contrôle (sans stress).

### Mesure des acides gras à chaines courtes :

Les mesures ont été réalisées en phase stationnaire de croissance des souches. Nous avons récupéré les surnageants et les avons stockés à -20°C jusqu'à la détermination des acides gras à chaine courte (AGCC).

Les surnageants bactériens ont été déprotéinés pendant une nuit à 4°C via l'ajout d'acide phosphotungstique à 10 % [p/v] (acide phosphotungstique, Sigma).

Nous avons ensuite centrifugé les échantillons résultants pendant 15 min à 12 000 g.

Les concentrations d'AGCC ont été déterminées à l'aide d'un chromatographe en phase gazeuse (GC ; Agilent 6890 N Network) équipé d'un injecteur split-splitless (GC Ag-ilent 7890B), d'un détecteur à ionisation de flamme et d'une colonne capillaire remplie de SP 1000 (Nukol ; Supelco 25 236).

Le débit d'hydrogène, gaz porteur, était de 10 mL/min ; les températures de l'injecteur, de la colonne et du détecteur étaient respectivement de 200°C, 100°C et 240°C.

Nous avons utilisé le 2-éthylbutyrate comme étalon interne dans nos échantillons et avons utilisé un panel d'étalons AGCC.

Deux répétitions ont été réalisées pour chaque échantillon. Nous avons collecté les données AGCC et intégré les pics à l'aide du logiciel par défaut du GC (Agilent).

Pour déterminer les concentrations finales des AGCC, les surnageants ont été pesés avant et après la précipitation des protéines afin d'obtenir le facteur de multiplication approprié (c'est-à-dire le rapport de masse du surnageant sur l'échantillon).

### Mesure de résistance aux antibiotiques :

La mesure de la résistance aux antibiotiques a été menée par la méthode E-Test.

Des suspensions bactériennes ont été réalisées dans de l'eau peptonée stérile avec des colonies fraîches pour obtenir une unité McFarland de 0,5.

Les suspensions ont été étalées sur des plaques pour obtenir un tapis bactérien, avec un écouvillon stérile (Biomerieux).

Des bandelettes d'antibiotiques (E-test, BioMérieux) ont été ajoutées sur des plaques, suivies d'une incubation de 48 heures à 37°C.

La zone d'inhibition avec la concentration correspondante (concentration minimale inhibitrice, CMI) a ensuite été déterminée et comparée aux lignes directrices de l'EFSA « *Guidance on the caractérisation of microorganisms used as feed additives or* as *production organismes »,* 2018).

### Evaluation d'activité DNAse :

L'activité de la DNase a été mesurée en étalant chaque souche bactérienne d'une culture en MRSc sur la nuit, sur une gélose de DNase test contenant du vert de méthyle.

L'activité positive a été prise en compte lorsque le support changeait de couleur ou qu'un halo transparent se formait.

### Activité anti-inflammatoire des souches in vitro :

Les profils immunomodulateurs ont été étudiés par quantification de la cytokine pro-inflammatoire inter-leukine-8 (IL-8) après une co-incubation de bactéries avec des cellules HT-29.

Tout d'abord, nous avons ensemencé des cellules HT-29 dans des plaques à 24 puits (1 × 10⁵ cellules par puits).

Au bout de six jours, la confluence a été atteinte et le milieu complet a été remplacé par un milieu DMEM glutaMAX contenant 5 % de FBS.

Après 24 h, nous avons remplacé ce milieu par du DMEM glutaMAX, 0,1% de pénicilline/streptomycine (P/S ; Sigma), 5% de FBS et complété ou non par du TNF-α à une concentration finale de 5 ng/mL (TNF-α, Peprotech) auxquelles nous avons ajouté des cellules bactériennes à multiplicité d'infection (MOI) de 40.

Après 6 heures de co-incubation, les surnageants des cultures de cellules eucaryotes ont été récupérés et stockés à -80°C. Les concentrations d'IL-8 ont été quantifiées à l'aide d'un kit Human IL-8 (Human IL-8 ELISA MAX Standard Set, BioLegend).

L'absorbance a été mesurée à 450 nm à l'aide d'Infinite M200 pro (TECAN).

### Analyse statistique

Pour évaluer les propriétés anti-inflammatoires, un test paramétrique de L'ANOVA à un facteur été appliqué, suivi d'un test post hoc de Fischer sans correction pour comparaisons multiples afin d'analyser les différences entre les traitements, à l'aide du logiciel GraphPad Prism (version 10.4.1, La Jolla, CA, USA). Les valeurs sont exprimées en moyenne ± ESM.

### 2. Résultats et Analyse

Les souches isolées des selles de nourrissons ont été caractérisées pour répondre aux prérequis d'utilisation à travers des compléments alimentaires.

Ainsi, il a été déterminé leur résistance aux conditions gastriques, à savoir la présence de sels biliaires et de pH acide auxquelles elles sont exposées lors de l'ingestion par voie orale.

En moyenne, les souches appartenant au genre *Limosilactobacillus* ont présenté une bonne survie au pH acide avec une perte de 0,046 log à 0,32 log (ce qui représente 0,9x10^9 à 0,5x10^9 en UFC pour un traitement contenant 1x10^9 UFC, approximativement 90,9-47,89% en cellules viables).

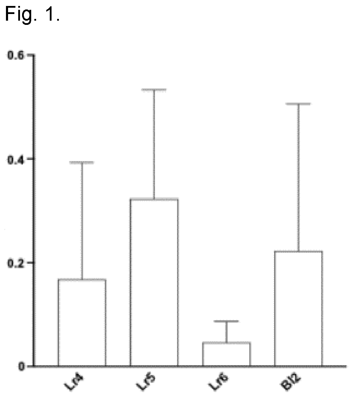

Pour la souche *B*. *longum sbsp. longum* une perte de de 0,22 log a été détectée (ce qui représente 0,610^9 en UFC pour un traitement contenant 1x10^9 UFC, 60,22% en cellules viables) (Figure 1).

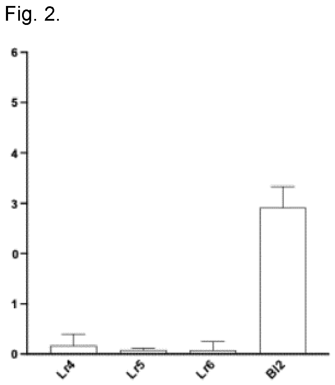

La souche appartenant aux genres *Bifidobacterium* a une moindre résistance aux acides biliaires, avec une perte de viabilité supérieure à 2 log, (ce qui représente 1,22×10^6 en UFC pour un traitement contenant 1x10^9 UFC, 0,12% en cellules viables) mais les souches appartenant au genre *Limosilactobacillus* ont une bonne survie avec une perte de seulement 0,07 à 0,16 log (ce qui représente 8,54x10^8 à 6,92x10^8 en UFC pour un traitement contenant 1x10^9 UFC, approximativement 85,47- 69,2% en cellules viables) (Figure 2).

Par ailleurs, nous avons mesuré la quantité d'AGCC sécrétés par les souches. L'ensemble des souches a été capable de sécréter de l'acétate avec des valeurs variant de 14,63 à 48 µmol/g avec cependant une plus forte production pour les bifides (Tableau 2).

La résistance aux antibiotiques a été évaluée par E-test. Les souches Lr4, Lr5 et Lr6 ont une résistance à l'ampicilline mais ne présentent pas d'autres résistances à l'exception de la souche Lr4 pour la tétracycline (Tableau 3).

La souche *B*. *longum sbsp. longum* est sensible aux antibiotiques.

Toutes les souches ont montré l'absence d'activité DNASE.

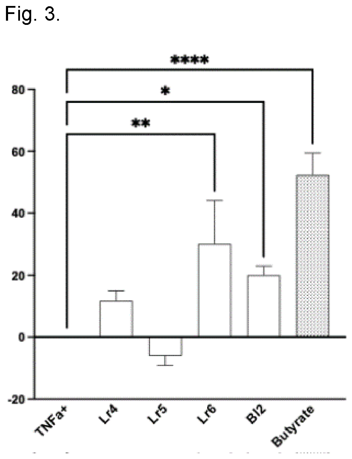

En parallèle, nous avons défini comme prérequis la validation de propriété anti-inflammatoires. Seules les souches Lr6, et BI2 ont diminué significativement de 30% et 20% le niveau d'IL8 induit par le TNF-a et ont donc présenté une activité anti-inflammatoire (Figure 3).

**[Tableau 2]**

| Code | Acétate (µm/g) |
|---|---|
| Lr4 | 26,6 ± 7,02 |
| Lr5 | 14,63 ± 0,98 |
| Lr6 | 23,27 ± 14,38 |
| BI2 | 47,95 ± 14,96 |

### Mesure de la production d'acides gras à courte chaine

**[Tableau 3]**

| Code | Ampi | Vanco | Genta | Kana | Erythro | Strepto | Clinda | Tetra | Chloram | Cipro | Coli | Fosfo |
|---|---|---|---|---|---|---|---|---|---|---|---|---|
| Lr4 | R | NR | S | S | S | S | S | R | S | NR | NR | NR |
| Lr5 | R | NR | S | S | S | S | S | S | S | NR | NR | NR |
| Lr6 | R | NR | S | S | S | S | S | S | S | NR | NR | NR |
| BI2 | S | S | S | NR | S | S | S | S | S | NR | NR | NR |

| | | | | | | | | | | | | |
|---|---|---|---|---|---|---|---|---|---|---|---|---|
| Profile de résistance aux antibiotiques Légende : S sensible, R résistance selon les seuils établis pour les espèces cibles, NR non requis. | | | | | | | | | | | | |

Sur la base des résultats selon l'Exemple 1, 2 souches ont été sélectionnées pour être testées *in vivo* dans un modèle de césarienne (Lr4 et BI2, correspondent respectivement à CNCM I-6110 et I-6114) et deux dans un modèle de challenge précoce aux antibiotiques (Lr5 et Lr6, correspondent respectivement à CNCM I-6111 et I-6112).

### Exemple 2 - Évaluation de la protection contre les effets physiologiques d'une naissance par césarienne in vivo

### 1. Matériel et Méthode

Des souriceaux nés par voie basse et par césarienne ont été répartis selon les groupes suivant :
- Animaux contrôles nés par voie basse + PBS
- Animaux contrôles nés par césarienne + PBS
- Animaux nés par césarienne + Lr4 (CNCM I-6110)
- Animaux nés par césarienne + BI2 (CNCM I-6114)

Les souriceaux ont reçu par tétage soit du PBS (agent véhicule contrôle), soit les bactéries Lr4 (CNCM I-6110) ou BI2 (CNCM I-6114) contenant 1x10^9 UFC/j à J1 et J3 de vie.

Les animaux ont été suivi et autopsiés après 1 semaine, 3 semaines (sevrage) et 6 semaines. Une partie des animaux a été challengée au DNBS par voie intrarectale avec 200 mg/kg dans 50 µl de PBS éthanol 30% (EtOH) pour mimer une colite en semaine 6.

Parmi les analyses effectuées, un suivi de poids, la mesure d'un marqueur systémique de perméabilité (sCD14, Elisa R&D systems), l'épaisseur de la paroi intestinale via un paquimètre de précision afin d'évaluer les changements structuraux en lien avec l'inflammation colique, l'inflammation systémique (lipocaline 2, LPN-2), du score macroscopique (après challenge au DNBS), l'inflammation intestinale (épaisseur de la paroi colique) la quantité de cellules positives au bleu alcian (AB+) par crypte intestinale (pour vérifier le développement fonctionnel du côlon), l'activité systémique antioxydante totale, la mesure du percentage de cellules T régulatrices (CD3+CD4+FOXP3+) parmi les populations de cellules T(CD3+CD4+) et de cellule macrophages (CD11b+MHCII+F4/80+) parmi les cellules myéloïdes (CD11b+MHCII+) afin d'évaluer l'empreinte immunitaire ont été réalisées. Le changement de poids après challenge au DNBS a été exprimé en aire sous la courbe de la différence entre le poids attendu et le poids observés (Barone M et al. 2023, Microbiome ; doi: 10.1186/s40168-023-01584-0).

### Analyse statistique

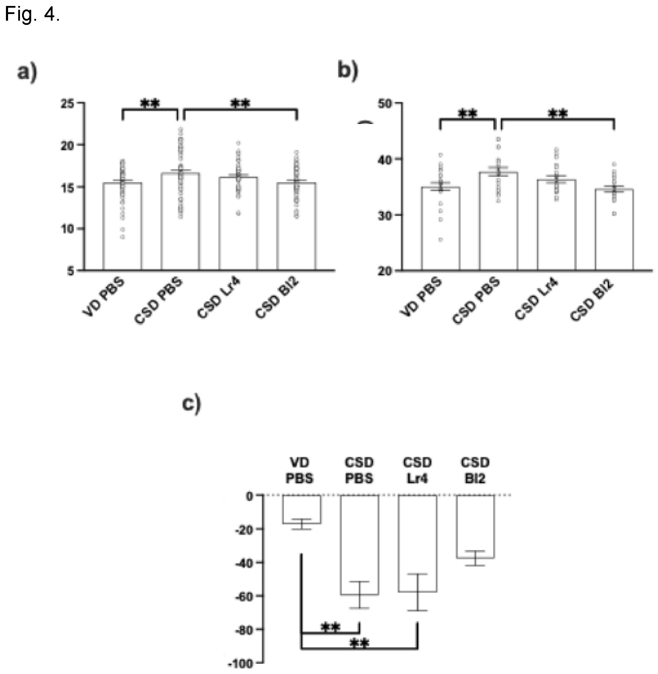

Pour évaluer les différences entre les traitements pour le poids corporel des animaux (Fig. 4a-b), l'aire sous la courbe des variations de poids corporel induites par le DNBS (poids corporel réel vs attendu ; Fig. 4c), les niveaux plasmatiques de sCD14 et de LPN2 (Fig. 5a-b) ainsi que l'activité antioxydante totale et les cellules immunitaires T régulatrices (Fig. 6b-c), un test paramétrique d'ANOVA à un facteur a été appliqué, suivi d'un test post hoc de Fisher sans correction pour comparaisons multiples afin d'analyser. Une analyse à effets mixtes, suivie d'un test post hoc de Fisher sans correction pour comparaisons multiples, a été réalisée pour évaluer les différences des niveaux plasmatiques de sCD14 (Fig. 5c) et de l'épaisseur de la paroi colique (Fig. 5d) entre les groupes âgés de 6 semaines, avec ou sans challenge au DNBS. Aucun effet du sexe n'a été observé dans les analyses mentionnées ci-dessus. Enfin, les différences entre les groupes pour les cellules positives au bleu Alcian par crypte intestinales et les cellules macrophagiques (Fig. 6 a,d) ont été évaluées à l'aide d'un test Kruskal-Wallis, suivi d'un test post hoc de Dunn sans correction pour comparaisons multiples. Les analyses statistiques ont été réalisées à l'aide du logiciel GraphPad Prism (version 10.4.1, La Jolla, CA, USA). Les valeurs sont exprimées en moyenne ± ESM.

### 2. Résultats

Les animaux nés par césarienne ont un poids plus élevé en semaine 3 et semaine 6 de vie *versus* ceux nés par voie basse et les animaux supplémentés avec la souche BI2 ont un profil proche des animaux nés par voie basse en semaine 3 et en semaine 6 seulement pour les mâles (Figure 4a-b). De plus, après injection au DNBS, la perte de poids (réelle vs. prédite) est plus marquée chez les animaux nés par césarienne versus ceux nés par voie basse mais cette perte n'est pas significative pour les animaux supplémentés avec la souche Lr4 suggérant un moindre impact chez ces animaux (Figure 4c).

De même, les animaux nés par césarienne présentent en semaine 3 une élévation du CD14 sérique ainsi qu'en semaine 6 après challenge au DNBS *versus* les animaux nés par voie basse (Figure 5a et c). A nouveau, la supplémentation avec la souche BI2 améliore ce paramètre en semaine 3 et les deux souches Lr4 et BI2 améliorent cette augmentation de la perméabilité après induction au DNBS (Figure 5b-c). Le DNBS induit par ailleurs une augmentation de l'épaisseur du colon (marqueur d'inflammation) mais les deux souches BI2 et Lr4 diminuent cette augmentation chez les animaux nés par césarienne (Figure 5d).

Dans la première semaine de vie, une diminution de la quantité dans les cellules caliciformes positives à la mucine acide par crypte colique est induite par la césarienne. BI2 permet de rétablir ce paramètre à des niveaux équivalents à ceux des animaux contrôles (Figure 6a). La césarienne induit une réponse compensatrice en augmentant l'activité antioxydante totale en semaine 3, cet effet n'est pas visible avec la supplémentation des animaux par les souches Lr4 et BI2 qui présentent une diminution de cette activité compensatrice (Figure 6b). De plus, les animaux nés par césarienne ont une augmentation des cellules T régulatrices (CD3+CD4+FOXP3+) dans les ganglions mésentériques (Figure 6c). Ceci est diminué avec la supplémentation par la souche Lr4. Enfin, en semaine 6, le traitement au DNBS induit une augmentation des cellules macrophagiques (CD11b+MHCII+F4/80+) au niveau des ganglions mésentériques chez les animaux nés par césarienne (Figure 6d). Cependant, la supplémentation avec la souche BI2 permet de réduire cette augmentation à des niveaux équivalents aux animaux contrôles.

### Exemple 3 - Évaluation de la protection contre les effets physiologiques d'une prise antibiotique précoce in vivo

### 1. Matériel et Méthode

Des souriceaux nés par voie basse ont été répartis selon les groupes suivant :
- Animaux contrôles sans antibiotiques (sans ATB) + PBS
- Animaux contrôles exposés aux antibiotiques (ATB) + PBS
- Animaux exposés aux antibiotiques (ATB) + LR5 (CNCM I-6111)
- Animaux c exposés aux antibiotiques (ATB) + LR6 (CNCM I-6112)

Les souriceaux ont reçu par tétage soit du PBS (agent véhicule contrôle), soit les bactéries Lr5 et Lr6 (1x10⁹CFU/j) pendant 4 semaines dont 2 par tétage puis par gavage orale.

Le cocktail antibiotiques (néomycine, amoxicilline et métronidazole à 0,5 mg/ml chacun) a été administré dans l'eau de boisson du jour J14 de grossesse jusqu'à 4 semaine de vie des souriceaux. Les antibiotiques administrés en début de vie ont un impact sur le développement des souris.

Les animaux ont été suivi et autopsiés en semaine 4 et 6 semaines. Une partie des animaux a été challengée au DNBS pour mimer une colite en semaine 6.

Parmi les analyses effectuées, un suivi de poids, de longueur du corps, du colon, et de l'intestine grêle a été utilisé comme indicateurs du développement normal, la mesure de l'inflammation systémique (lipocaline 2, LPN-2), et du score macroscopique (après challenge au DNBS), la mesure des taux de cytokines pro-inflammatoires coliques du facteur de nécrose tumorale (TNF)-α et de l'épaisseur, de la longueur du côlon (pour évaluer l'inflammation locale) et de la myélopéroxydase iléale (MPO, un marqueur de l'activation des neutrophiles) ont été réalisées. Le traitement antibiotique a entraîné une augmentation significative de la colite, ce qui a impacté la survie des animaux face à ce challenge. De cette manière, nous avons également apporté des données sur l'effet des souches sélectionnées sur le pourcentage de survie.

### Analyse statistique

Le test Log-rank (Mantel-Cox) a comparé les courbes de survie entre PBS, Lr5 et Lr6 traitements (Fig. 8a) et les valeurs sont exprimées fraction de survie ± erreur standard (ES).

L'analyse des différences pour la longueur du colon et le niveau plasmatique de LPN 2 entre les groupes avec des animaux âgés de 4 semaines (Fig.7a-b), pour le poids corporel, la longueur de l'intestin grêle et du score macroscopique (Fig. 8b-d), pour la mesure du TNF-α et la longueur du colon (Fig. 9 a) pour les animaux âgés de 6 semaines a été réalisée à l'aide d'un test ANOVA à un facteur, suivi d'un test post hoc de Fisher sans correction pour comparaisons multiples. Pour les animaux âgés de 6 semaines, le poids corporel et la longueur corporelle (Fig. 7c-d et Fig. 8), ont été analysé avec une analyse à effets mixtes, suivie d'un test post hoc de Fisher sans correction pour comparaisons multiples, a été effectuée. Pour les animaux âgés de 6 semaines, l'épaisseur du colon et la MPO iléale ont été analysée avec un test Kruskal Wallis suivi d'un test post hoc de Dunn sans corrections multiples (Fig. 9b-d). Les *valeurs sont exprimées en moyenne* ± *ESM.* Statistiques ont été réalisées à l'aide du logiciel GraphPad Prism (version 10.4.1, La Jolla, CA, USA).

### 2. Résultats et Analyse

Lors des essais de challenge précoce aux antibiotiques, il a été noté une réduction de l'inflammation colique caractérisée par une augmentation de la taille du colon chez les souriceaux femelles en semaine 4 de vie pour ceux supplémentés avec la souche Lr6 (Figure 7a). De plus les souriceaux ayant été supplémentés avec les souches Lr5 et Lr6 ont une diminution de l'inflammation systémique (Figure 7b). Les souriceaux ayant été supplémentés par la souche Lr5 ont un poids corporel augmenté (Figure 7c), ainsi qu'une augmentation de la longueur corporelle (Figure 7d) par rapport aux animaux contrôles traités par antibiotiques en semaine 6. Une récupération partielle de la perte de longueur de l'intestin grêle a été induite par la souche Lr5, sans être perturbée par le modèle de colite (Fig. 8c).

Les souriceaux traités précocement par antibiotiques et challengés en semaine 6 par du DNBS pour induire une colite ont taux de survie réduit à 70% contre 100% de survie pour les souriceaux supplémentés par la souche Lr6 et supérieure à 94% pour la souche Lr5 (Figure 8a). Malgré la perte d'animaux, nous avons observé que la souche Lr6 a réduit la perte de poids corporel induite par la colite (Fig. 8b) et a également diminué les altérations structurelles macroscopiques induites par le modèle d'inflammation colique (Fig. 8d).

En semaine 6, les animaux sous antibiotiques (AEL PBS EtOH) ont une augmentation du TNF-α au niveau du colon. La supplémentation des animaux avec la souche Lr5 permet d'empêcher cet effet avec des niveaux comparables à ceux présents chez les animaux contrôles PBS (Figure 9a). Après challenge au DNBS en Semaine 6, les animaux supplémentés avec la souche Lr6 ont une amélioration de la longueur et de l'épaisseur du colon ainsi que qu'une diminution de la MPO au niveau de l'iléon (Figure 9 b-d).

## Revendications

1. Bactéries pour leur utilisation chez un enfant et/ou sa mère, au moins au cours de la période allant de la naissance dudit enfant jusqu'aux deux ans révolus dudit enfant, dans la prévention ou le traitement des effets délétères chez ledit enfant résultants de maladies non transmissibles qui sont observées, à moyen et/ou long termes, après une dysbiose du microbiote en âge précoce (correspondant à la primo-colonisation),
lesquelles bactéries sont choisies parmi les souches primo-colonisatrices d'un microbiote non dysbiotique, appartenant à un genre choisi parmi :
- *Bifidobacterium,* de préférence parmi *Bifidobacterium longum,*
- *Limosilactobacillus,* par exemple parmi *Limosilactobacillus reuteri,*
lesquelles bactéries présentent les propriétés suivantes :
- une réduction de l'inflammation intestinale, mesurée in vitro par dosage de la cytokine inflammatoire IL-8 sur lignée épithéliale intestinale humaine,
- une production d'acétate,
et, de préférence encore :
- une résistance au pH acide,
- une résistance aux sels biliaires,
- une absence d'activité DNASE,
- une absence d'antibiorésistance.

2. Bactéries selon la revendication 1, **caractérisées en ce que** lesdites bactéries sont isolées parmi les souches présentes dans les selles de nourrissons, lesdits nourrissons étant nés par voie basse et allaités, non médicamentés (en particulier en absence d'antibiotiques) et ayant moins de 3 jours, et dont leurs mères ne présentaient pas de maladie chronique, un IMC normal et n'ont pas été médicamentées, ni supplémentées en probiotiques le dernier trimestre de grossesse.

3. Bactéries selon l'une quelconque des revendications 1 ou 2, caractérisées que lesdites bactéries sont choisies parmi les bactéries disposant d'un statut Présomption d'innocuité reconnue (QPS).

4. Bactéries selon l'une quelconque des revendications 1 à 3, **caractérisées en ce que** ladite période s'étend sur les 1000 premiers jours de l'enfant, depuis la grossesse jusqu'au deux ans révolus de l'enfant.

5. Bactéries selon l'une quelconque des revendications 1 à 4, **caractérisées en ce que** lesdites bactéries appartiennent aux souches choisies parmi :
- la souche de l'espèce *L. reuteri* déposée auprès de la CNCM sous le numéro d'accession CNCM I-6110 le 11 septembre 2024,
- la souche de l'espèce *L. reuteri* déposée auprès de la CNCM sous le numéro d'accession CNCM I-6111 le 11 septembre 2024,
- la souche de l'espèce *L. reuteri* déposée auprès de la CNCM sous le numéro d'accession CNCM I-6112 le 11 septembre 2024,
- la souche de l'espèce *B. longum sbsp. longum* déposée auprès de la CNCM sous le numéro d'accession CNCM I-6114 le 11 septembre 2024.

6. Bactéries selon l'une quelconque des revendications 1 à 5, **caractérisées en ce que** lesdites maladies non transmissibles sont choisies parmi les maladies chroniques, de préférence encore parmi :
- les maladies inflammatoires chroniques de type MICI (maladie inflammatoire chronique de l'intestin),
- les maladies métaboliques, ou
- les allergies.

7. Bactéries selon l'une quelconque des revendications 1 à 6, **caractérisées en ce que** ladite dysbiose est survenue en âge précoce (de préférence dans une fenêtre des 1000er jours), éventuellement suite à au moins un facteur choisi parmi :
- une naissance par voie césarienne,
- la prise d'antibiotiques, ou
- un allaitement artificiel.

8. Composition, par exemple sous la forme d'un complément alimentaire, d'une Denrée Alimentaire Destinée à des Fins Médicales Spéciales ou d'un produit infantile, comprenant des bactéries selon l'une quelconque des revendications 1 à 7, de préférence vivante,
laquelle composition comprend de préférence au moins un ingrédient additionnel choisi parmi :
- une vitamine D,
- un prébiotique,
- les oligosaccharides du lait maternel (HMOs).

9. Composition selon la revendication 8, **caractérisée en ce que** ladite composition se présente sous une forme adaptée à l'administration par voie orale chez l'enfant, au cours de la période périnatale, par exemple sous forme de gouttes ou de sachets à diluer.

10. Composition selon l'une quelconque des revendications 8 ou 9, sous forme d'un complément alimentaire, adaptée à une utilisation non-thérapeutique au cours de la période périnatale chez l'enfant et/ou la mère, dans la prévention ou le traitement des effets délétères observés à moyen et/ou long termes suite à une dysbiose du microbiote en âge précoce (correspondant à la primo-colonisation).

11. Procédé pour la sélection de bactéries selon l'une quelconque des revendications 1 à 7,
lequel procédé de sélection comprend les étapes successives suivantes :
- une étape de collecte, au cours de laquelle des selles de nourrissons sont collectés, lesdits nourrissons étant nés par voie basse et allaités, non médicamentés (en particulier en absence d'antibiotiques) et ayant moins de 3 jours, et dont leurs mères ne présentaient pas de maladie chronique, un IMC normal et n'ont pas été médicamentées, ni supplémentées en probiotiques le dernier trimestre de grossesse,
- une étape de sélection, au cours de laquelle ladite au moins une souche bactérienne est isolée et sélectionnée dans lesdits selles de nourrissons.
